**Europäisches Patentamt**

**European Patent Office**

**Office européen des brevets**

(19)

(11) Veröffentlichungsnummer: **0 273 137**
**B1**

# EUROPÄISCHE PATENTSCHRIFT

(12)

(45) Veröffentlichungstag der Patentschrift:
**27.12.90**

(21) Anmeldenummer: **87116065.1**

(22) Anmeldetag: **31.10.87**

(51) Int. Cl.⁵: **A61K 6/04, C22C 5/04**

(54) **Verwendung von Palladiumlegierungen zur Herstellung von Zahnersatz.**

(30) Priorität: **12.12.86 DE 3642474**

(43) Veröffentlichungstag der Anmeldung:
**06.07.88 Patentblatt 88/27**

(45) Bekanntmachung des Hinweises auf die Patenterteilung:
**27.12.90 Patentblatt 90/52**

(84) Benannte Vertragsstaaten:
**AT BE CH DE ES FR GB GR IT LI LU NL SE**

(56) Entgegenhaltungen:
EP-A- 0 124 750
DE-A- 3 247 398
DE-C- 3 239 338

(73) Patentinhaber: **Degussa Aktiengesellschaft,
Weissfrauenstrasse 9, D-6000 Frankfurt am Main 1(DE)**

(72) Erfinder: **Wagner, Rudolf, Dr. Dipl.-Ing., Breslauer
Strasse 10, D-7537 Remchingen(DE)**
Erfinder: **Stümke, Manfred, Dr. Dipl.-Phys., Am
Waldweg 21, D-7530 Pforzheim(DE)**
Erfinder: **Groll, Werner, Dr. Dipl.-Ing., Gartenstrasse 5,
D-8755 Alzenau-Hörstein(DE)**
Erfinder: **Schiwiora, Harry, Theodor-Heuss-Strasse 37,
D-7530 Pforzheim(DE)**

## Beschreibung

Die Erfindung betrifft die Verwendung von Palladiumlegierungen zur Herstellung von festsitzendem und herausnehmbaren Zahnersatz.

Aus der DE-PS 33 16 595 sind Palladiumlegierungen zur Herstellung von festsitzendem und herausnehmbaren Zahnersatz bekannt, die 65 bis 85 % Palladium, 0 bis 10 % Gold und/oder 0 bis 5 % Platin, 0,1 bis 10 % Zinn, 1 bis 10 % Gallium, 1 bis 12 % Kupfer, sowie 0,05 bis 1,5 % Ruthenium und/oder 0,05 bis 0,7 % Rhenium enthalten. Der Nichtedelmetallgehalt kann also bis zu 32 Gew. % betragen.

Palladiumlegierungen mit solch hohen Unedelmetallanteilen neigen beim Aufschmelzen in oxidierender Atmosphäre mitunter zur Ausbildung einer auf der flüssigen Schmelze schwimmenden, recht stabilen Oxidhaut. Die Ausbildung einer solchen Oxidhaut bewirkt die Anreicherung und Verbrennung bevorzugt oxidierender Legierungsbestandteile und kann somit beim mehrmaligen Umschmelzen der Legierung zu einer ungewollten Veränderung der Legierungszusammensetzung führen. Weiterhin kann der Zahntechniker beim Erschmelzen solcher Legierungen in oxidierender Atmosphäre durch die oberflächliche Oxidbildung nicht genau erkennen, ob die unter der Oxidschicht liegende Legierung bereits flüssig genug ist, um vergossen werden zu könne. Eine weitere unerwünschte Nebenerscheinung bei der Ausbildung einer Oxidhaut ist, daß der Schmelztiegel mitunter nicht vollständig ausfließt, so daß Schlackerückstände am Tiegelboden zurückbleiben.

Diese Oxidhäute treten vor allem bei Legierungen auf, die mehr als 10 % Unedelmetalle enthalten, je nach Art der zugesetzten Unedelmetalle.

Es war daher Aufgabe der vorliegenden Erfindung, eine Palladiumlegierung zur Herstellung von festsitzendem und herausnehmbaren Zahnersatz zu entwickeln, aufbauend auf einer Palladiumlegierung gemäß der DE-PS 33 16 595, die beim Schmelzen auch bei höheren Unedelmetallgehalten keine Oxidhäute in oxidierender Atmosphäre ausbildet.

Diese Aufgabe wird erfindungsgemäß gelöst durch die Verwendung einer Palladiumlegierung aus 65 bis 85 % Palladium, 0 bis 10 % Gold und/oder 0 bis 5 % Platin, 0,1 bis 10 % Zinn, 1 bis 10 % Gallium, 1 bis 12 % Kupfer, 0,05 bis 1,5 % Ruthenium und/oder 0,05 bis 0,7 % Rhenium, sowie 0,01 bis 4 % Wolfram und/oder 0,01 bis 4 % Aluminium und/oder 0,01 bis 4 % Zink.

Besonders bewährt haben sich Legierungen, die neben 65 bis 85 % Palladium, 0 bis 10 % Gold und/oder 0 bis 5 % Platin, 0,1 bis 10 % Zinn, 1 bis 10 % Gallium, 1 bis 12 % Kupfer, 0,05 bis 1,5 % Ruthenium und/oder 0,05 bis 0,7 % Rhenium noch 0,1 bis 3 % Wolfram und/oder 0,05 bis 2 % Aluminium und/oder 0,05 bis 2 % Zink enthalten.

Diese Legierungen bilden beim Aufschmelzen in oxidierender Atmosphäre überraschenderweise keine Oxidschicht auf der Schmelze aus.

Folgende Tabelle gibt die Zusammensetzung solcher Legierungen wieder.

Tabelle:

| | | | | | | | | | |
|---|---|---|---|---|---|---|---|---|---|
| Pd | 82,0 | 79,3 | 78,4 | 77,7 | 76,5 | 76,0 | 76,2 | 71,0 | 70,0 |
| Au | | 1,0 | | | 2,0 | 1,5 | 1,5 | 2,5 | 4,0 |
| Pt | | 1,0 | | | | 0,5 | 0,5 | 1,5 | 4,0 |
| Cu | 5,0 | 5,0 | 9,5 | 9,0 | 11,3 | 11,0 | 11,0 | 10,0 | 9,0 |
| Sn | 5,3 | 6,5 | 1,0 | 3,0 | 1,9 | 1,9 | 1,9 | 9,0 | 4,0 |
| Ga | 6,0 | 6,0 | 9,6 | 8,5 | 7,2 | 7,2 | 7,2 | 2,5 | 6,3 |
| Ru | | 0,8 | 0,6 | 0,8 | 0,8 | 0,8 | | | 0,5 |
| Re | 0,5 | | 0,2 | | | | 0,5 | 0,5 | 0,2 |
| W | 1,0 | 0,3 | 0,5 | 0,6 | 0,3 | 0,7 | 0,7 | 2,0 | 1,6 |
| Al | 0,1 | 0,2 | 0,2 | 0,4 | | 0,2 | | 0,6 | 0,3 |
| Zn | 0,2 | | | | | 0,2 | 0,5 | 0,4 | 0,1 |

## Patentansprüche

1. Verwendung einer Palladiumlegierung aus 65 bis 85 % Palladium, 0 bis 10 % Gold und/oder 0 bis 5 % Platin, 0,1 bis 10 % Zinn, 1 bis 10 % Gallium, 1 bis 12 % Kupfer, 0,05 bis 1,5 % Ruthenium und/oder 0,05 bis 0,7 % Rhenium, sowie 0,01 bis 4 % Wolfram und/oder 0,01 bis 4 % Aluminium und/oder 0,01 bis 4 % Zink zur Herstellung von festsitzendem und herausnehmbaren Zahnersatz.

2. Verwendung einer Palladiumlegierung aus 65 bis 85 % Palladium, 0 bis 10 % Gold und/oder 0 bis 5 % Platin, 0,1 bis 10 % Zinn, 1 bis 10 % Gallium, 1 bis 12 % Kupfer, 0,05 bis 1,5 % Ruthenium und/oder 0,05 bis 0,7 % Rhenium, sowie 0,1 bis 3 % Wolfram und/oder 0,05 bis 2 % Aluminium und/oder 0,05 bis 2 % Zink für den Zweck gemäß Anspruch 1.

## Claims

1. The use of a palladium alloy of 65 to 85% palladium, 0 to 10% gold and/or 0 to 5% platinum, 0.1 to 10% tin, 1 to 10% gallium, 1 to 12% copper, 0.05 to 1.5% ruthenium and/or 0.05 to 0.7% rhenium and also 0.01 to 4% tungsten and/or 0.01 to 4% aluminium and/or 0.01 to 4% zinc for making permanent or removable false teeth.

2. The use of a palladium alloy of 65 to 85% palladium, 0 to 10% gold and/or 0 to 5% platinum, 0.1 to 10% tin, 1 to 10% gallium, 1 to 12% copper, 0.05 to 1.5% ruthenium and/or 0.05 to 0.7% rhenium and also 0.1 to 3% tungsten and/or 0.05 to 2% aluminium and/or 0.05 to 2% zinc for the purpose claimed in claim 1.

## Revendications

1. Utilisation d'un alliage de palladium à base de 65 à 85% en palladium, de 0 à 10% d'or et/ou de 0 à 5% de platine, de 0,1 à 10% d'étain, de 1 à 10% de gallium, de 1 à 12% de cuivre, de 0,05 à 1,5% de ruthénium et/ou de 0,05 à 0,7% de rhénium ainsi que de 0,01 à 4% de tungstène et/ou de 0,01 à 4% d'aluminium et/ou de 0,01 à 4% de zinc pour l'obtention de prothèses de dents implantables et amovibles.

2. Utilisation d'un alliage de palladium à base de 65 à 85% de palladium, de 0 à 10% d'or et/ou de 0 à 5% de platine, de 0,1 à 10% d'étain, de 1 à 10% de gallium, de 1 à 12% de cuivre, de 0,05 à 1,5% de ruthénium et/ou de 0,05 à 0,7% de rhénium, ainsi que de 0,1 à 3% de tungstène et/ou de 0,05 à 2% d'aluminium et/ou de 0,05 à 2% de zinc pour l'objectif conformément à la revendication 1.